(19) 

Europäisches Patentamt
European Patent Office
Office européen des brevets

(11) **EP 4 046 163 B1**

(12) **EUROPEAN PATENT SPECIFICATION**

(45) Date of publication and mention
of the grant of the patent:
**12.07.2023 Bulletin 2023/28**

(21) Application number: **20789611.9**

(22) Date of filing: **13.10.2020**

(51) International Patent Classification (IPC):
*G16H 20/40* (2018.01)   *G16H 40/63* (2018.01)
*A61M 1/16* (2006.01)

(52) Cooperative Patent Classification (CPC):
**A61M 1/1613; G16H 20/40; G16H 40/63**

(86) International application number:
**PCT/EP2020/078807**

(87) International publication number:
**WO 2021/074168 (22.04.2021 Gazette 2021/16)**

(54) **DETERMINING INTERNAL FILTRATION RATE WITHIN A CAPILLARY HEMODIALYZER**

BESTIMMEN DER INTERNEN FILTRATIONSRATE IN EINEM HOHLFASERDIALYSATOR

DÉTERMINER LE TAUX DE FILTRATION INTERNE DANS UN DIALYSEUR À FIBRES CREUSES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.10.2019 EP 19202905**

(43) Date of publication of application:
**24.08.2022 Bulletin 2022/34**

(73) Proprietor: **Gambro Lundia AB
226 43 Lund (SE)**

(72) Inventors:
• **DONATO, Danilo
87036 Rende-Cosenza (IT)**
• **STORR, Markus
70794 Filderstadt (DE)**

(74) Representative: **Perchenek, Nils
Gambro Dialysatoren GmbH
Legal and Intellectual Property
Holger Crafoord-Strasse 26
72379 Hechingen (DE)**

(56) References cited:
**WO-A1-2018/188973   JP-A- 2005 131 123
US-A1- 2014 305 869**

• **LEGALLAIS C ET AL: "A theoretical model to predict the in vitro performance of hemodiafilters", JOURNAL OF MEMBRANE SCIENCE, vol. 168, no. 1-2, 15 April 2000 (2000-04-15), pages 3-15, XP055683643, NL ISSN: 0376-7388, DOI: 10.1016/S0376-7388(99)00297-5**
• **DONATO D ET AL: "Optimization of dialyzer design to maximize solute removal with a two-dimensional transport model", JOURNAL OF MEMBRANE SCIENCE, ELSEVIER BV, NL, vol. 541, 11 July 2017 (2017-07-11), pages 519-528, XP085187080, ISSN: 0376-7388, DOI: 10.1016/J.MEMSCI.2017.07.018**
• **RAFF M ET AL: "Advanced modeling of highflux hemodialysis", JOURNAL OF MEMBRANE SCIENCE, ELSEVIER BV, NL, vol. 216, no. 1-2, 1 May 2003 (2003-05-01) , pages 1-11, XP004422225, ISSN: 0376-7388, DOI: 10.1016/S0376-7388(02)00552-5**
• **LEE J C ET AL: "Mathematical analysis for internal filtration of convection-enhanced high-flux hemodialyzer", COMPUTER METHODS AND PROGRAMS IN BIOMEDICINE, ELSEVIER, AMSTERDAM, NL; US NATIONAL LIBRARY OF MEDICINE (NLM), BETHESDA, MD, US, vol. 108, no. 1, 1 October 2012 (2012-10-01), pages 68-79, XP002746544, ISSN: 0169-2607, DOI: 10.1016/J.CMPB.2012.01.001 [retrieved on 2012-02-10]**

EP 4 046 163 B1

**(Cont. next page)**

- **SAKIYAMA R ET AL: "Effect of blood flow rate on internal filtration in a high-flux dialyzer with polysulfone membrane", JOURNAL OF ARTIFICIAL ORGANS ; THE OFFICIAL JOURNAL OF THE JAPANESE SOCIETY FOR ARTIFICIAL ORGANS, SPRINGER-VERLAG, TO, vol. 15, no. 3, 26 April 2012 (2012-04-26) , pages 266-271, XP035109884, ISSN: 1619-0904, DOI: 10.1007/S10047-012-0643-7**

**EP 4 046 163 B1**

**Description**

**Technical Field**

The present disclosure relates to a method and a device for determining an internal filtration rate IFR within a capillary hemodialyzer.

**Description of the Related Art**

The difficult removal of middle molecules from patients suffering from end-stage kidney disease represents one of the major challenges of hemodialysis. High concentrations of middle molecular weight (MW) solutes, such as $\beta_2$-micro-globulin, myoglobin, complement factor D, and polyclonal free light chains ($\kappa$-FLC and $\lambda$-FLC), have been correlated to critical clinical outcomes in chronic kidney disease patients. In conventional hemodialysis, low MW solutes (e.g. urea and creatinine) are effectively removed by diffusion, whereas the removal of middle MW solutes is generally achieved with the superimposition of convective transport over pure diffusion. The use of more water-permeable high-flux dialyzers and hemodiafiltration to increase convective transport has been shown to enhance the clearance of middle MW solutes, but still leads to unsatisfactory clinical results.

In EP 3 102 312 B1 and EP 3 102 314 B1, a new class of membranes, defined as Medium cut-off (MCO), and hemodialyzers comprising such membranes are disclosed. The particular features of MCO membranes include high MW retention onset and MW cut-off value close to the molecular weight of albumin, allowing for the removal of solutes up to ca. 45 kDa with negligible albumin loss. The use of hemodialyzers equipped with MCO membranes has made it possible to perform a new therapy called expanded hemodialysis (HDx), in which convective and diffusive transport are efficiently combined to increase dialyzer removal capability over a wide range of molecular weights. This is due to the interplay among the larger permeability of the MCO membranes as compared to that of high-flux membranes, and their geometrical structure, which increase convective transport across the hemodialyzer membrane by enhancing internal filtration (i.e. movement of plasma water from the blood compartment towards the dialysate compartment across the porous membrane in the proximal part of the hemodialyzer) and back filtration (i.e. movement of plasma water from the dialysate compartment towards the blood compartment in the distal part of the hemodialyzer). The compensation of internal filtration (IF) with an adequate amount of back filtration (BF) also permits to avoid a complex set-up and the use of fluid reinfusion, thus overcoming some practical issues of hemodiafiltration.

Legallais, C. et al.: "A theoretical model to predict the in vitro performance of hemodiafilters", J. Membr. Sci. 168 (2000) 3-15, discloses a one-dimensional model for transport in hemodiafilters aimed at predicting their clearance spectrum based on module geometry, membrane transport properties and operating conditions. The model accounts for the dependence of mass transport coefficients on the actual flow conditions, concentration polarization of rejected species, and the dependence of the water filtration flux on the actual hydrostatic and osmotic pressure profiles along the dialyzer length.

Methods to experimentally quantify the rate of IF and BF applied to hemodialyzers equipped with MCO membranes have been recently described by A. Lorenzin et al.: "Quantification of internal filtration in hollow fiber hemodialyzers with Medium cut-off membrane", Blood Purif. 46 (2018) 196-204. However, the experimental quantification of IF generally requires complex settings, and it is not cost-effective. Therefore, a simple and effective method for determining internal filtration rate IFR within a hemodialyzer is still lacking.

**Summary**

The present disclosure provides a method and a device for determining an internal filtration rate IFR within a capillary hemodialyzer. The method only requires dimensions of the hemodialyzer, dimensions well as physical parameters of the hollow fiber membranes present in the hemodialyzer, and flow rates of blood and dialysate through the hemodialyzer, all of which are readily available.

**Brief Description of the Drawings**

Figure 1 shows a schematic perspective view and sectional detail views of a capillary hemodialyzer and fluid flows within the hemodialyzer;

Figure 2 shows a comparison of experimentally determined internal filtration rates and internal filtration rates determined according to the method of the present disclosure for one model of a hemodialyzer;

3

Figure 3    shows a comparison of experimentally determined internal filtration rates and internal filtration rates determined according to the method of the present disclosure for another model of a hemodialyzer.

**Detailed Description**

The present disclosure provides a computer-implemented method for determining an internal filtration rate IFR within a capillary hemodialyzer. The method comprises

i) acquiring, using a computer, data on physical properties of the hemodialyzer and of hollow fibers present in the hemodialyzer;
ii) acquiring, using the computer, a previously stored blood flow rate $Q_B$ and a dialysis flow rate $Q_D$ through the hemodialyzer relating to a past operation of the hemodialyzer;
iii) determining, using the computer, the internal filtration rate IFR of the hemodialyzer, based on the data acquired in steps i and ii.

In one embodiment, the method additionally comprises

iv) acquiring, using the computer, data on a duration TD of the operation of the hemodialyzer;
v) determining, using the computer, a total volume $V_{tot}$ of fluid exchanged through the wall of the hollow fiber membranes present in the hemodialyzer during operation of the hemodialyzer, based on the internal filtration rate IFR and the data acquired in step iv.

In a particular embodiment, data on physical properties of the hemodialyzer and of hollow fibers present in the hemodialyzer are acquired from a database in operative association with a processor of the computer.
In one embodiment of the method, the database comprises data on physical properties of a plurality of different hemodialyzers and of hollow fibers present in the hemodialyzers. For each hemodialyzer of the plurality of different hemodialyzers, such data may comprise a diameter $d_H$ of the housing of the hemodialyzer, a number N of the hollow fibers present in the hemodialyzer, an effective length L of the hollow fibers present in the hemodialyzer, a total surface area $A_{tot}$ of the hollow fibers present in the hemodialyzer, an internal diameter $d_B$ of the hollow fibers present in the hemodialyzer, a wall thickness $\delta_M$ of the hollow fibers present in the hemodialyzer, a porosity $\varepsilon_M$ of the hollow fibers present in the hemodialyzer, and an ultrafiltration coefficient $K_{UF}$ of the hollow fibers present in the hemodialyzer.
Additionally, the database may comprise data on a blood viscosity $\mu_B$ and a dialysate viscosity $\mu_D$.
In a further embodiment, data on physical properties of the hemodialyzer and of hollow fibers present in the hemodialyzer and/or the blood flow rate $Q_B$ and the dialysis flow rate $Q_D$ through the hemodialyzer during operation of the hemodialyzer is acquired from an input device in operative association with a processor of the computer.
The hemodialyzer is perfused with blood at a blood flow rate $Q_B$ and dialysate at a dialysate flow rate $Q_D$ during operation, i.e., when it is used in an external blood circuit to remove toxins from blood. The internal filtration rate IFR and the total volume $V_{tot}$ of fluid exchanged through the wall of the hollow fiber membranes present in the hemodialyzer vary with blood flow rate $Q_B$ and dialysate flow rate $Q_D$ through the hemodialyzer.
In one embodiment, the input device comprises a contactless reader. In a particular embodiment, the contactless reader is an optical reader which acquires data of a barcode or QR code present on a hemodialyzer. In another particular embodiment, the contactless reader is a sensor which acquires data from an RFID or NFC tag present on or in the hemodialyzer.
In a further embodiment, the input device comprises at least one user interface. In a particular embodiment, the user interface comprises a keyboard or a touchscreen. In a further embodiment of the system, the input device comprises a graphical use interface (GUI). In a particular embodiment, the input device comprises a touchscreen of a smartphone. The input device is used for manually entering data on physical properties of the hemodialyzer and of hollow fibers present in the hemodialyzer, and/or the blood flow rate $Q_B$ and the dialysis flow rate $Q_D$ through the hemodialyzer during operation of the hemodialyzer.
In another embodiment, the input device comprises an extracorporeal blood treatment apparatus. The extracorporeal blood treatment apparatus controls the parameters of an extracorporeal blood circuit comprising a hemodialyzer during operation, i.e. during the treatment of a patient. When used as an input device for the method of the present disclosure, the extracorporeal blood treatment apparatus provides data on physical properties of the hemodialyzer and of hollow fibers present in the hemodialyzer, and/or the blood flow rate $Q_B$ and the dialysis flow rate $Q_D$ through the hemodialyzer during operation of the hemodialyzer. In one embodiment of the method, the processor acquires real-time data of the blood flow rate $Q_B$ and the dialysis flow rate $Q_D$ through a hemodialyzer during operation of the hemodialyzer and, optionally, data on the duration TD of the operation, from the extracorporeal blood treatment apparatus, i.e. during an actual treatment. In another embodiment, a blood flow rate $Q_B$ and a dialysis flow rate $Q_D$ through a hemodialyzer during operation of the hemodialyzer and, optionally, a duration TD of the operation, are manually entered through the input device to simulate a treatment.

In one embodiment of the method, the data on physical properties of the hemodialyzer and of hollow fibers present in the hemodialyzer comprise a diameter $d_H$ of the housing of the hemodialyzer, a number N of the hollow fibers present in the hemodialyzer, an effective length L of the hollow fibers present in the hemodialyzer, a total surface area $A_{tot}$ of the hollow fibers present in the hemodialyzer, an internal diameter $d_B$ of the hollow fibers present in the hemodialyzer, a wall thickness $\delta_M$ of the hollow fibers present in the hemodialyzer, a porosity $\varepsilon_M$ of the hollow fibers present in the hemodialyzer, and an ultrafiltration coefficient $K_{UF}$ of the hollow fibers present in the hemodialyzer.

In one embodiment of the method, the internal filtration rate IFR is determined according to

$$IFR = N\pi d_B \int_0^{x_i} J_v(z)dz$$

with

N       number of hollow fibers present in the hemodialyzer;
$d_B$      internal diameter of hollow fibers present in the hemodialyzer;
$J_v(z)$    ultrafiltration flux through the membrane wall;
$x_i$      location of inversion point ($J_v(x_i) = 0$) .

This equation can be rewritten as

$$IFR = N\pi d_B K \int_0^{x_i} (P_B(z) - P_D(z) - \pi_o)dz$$

with

K    $-\dfrac{K_{UF}}{A_{tot}\,\varepsilon_M}$ , $K_{UF}$ ultrafiltration coefficient of the hollow fibers present in the hemodialyzer, $A_{tot}$ total membrane area of the hollow fibers present in the hemodialyzer ($A_{tot} = N\pi d_B L$), $\varepsilon_M$ membrane porosity of the hollow fibers present in the hemodialyzer;

$P_B(z)$   blood pressure;
$P_D(z)$   dialysate pressure;
$\Pi_o$     average oncotic pressure;

In another embodiment of the method, the internal filtration rate IFR is determined according to

$$IFR = N\pi d_B K \left\{ (P_{B,out} - P_{D,out} - \pi_0)x_i \right.$$

$$- \frac{128\mu_B}{N\pi d_B^4}\left[ Q_B\left(\frac{x_i^2}{2} - Lx_i\right) - N\pi d_B\left[ \frac{v_2 - v_1}{6L}\left(\frac{x_i^4}{4} - L^3 x_i\right) + \frac{v_1}{2}\left(\frac{x_i^3}{3} - L^2 x_i\right)\right]\right]$$

$$\left. + \frac{\mu_D}{2N\pi f_{R_o,R_k}}\left[ Q_D\frac{x_i^2}{2} - N\pi d_B\left[ \frac{v_2 - v_1}{2L}\left(\frac{x_i^4}{12} - \frac{L^2}{2}x_i^2\right) + v_1\left(\frac{x_i^3}{6} - \frac{L}{2}x_i^2\right)\right]\right]\right\}$$

with

N                          number of hollow fibers present in the hemodialyzer;
$d_B$                         internal diameter of the hollow fibers present in the hemodialyzer;

| K | $\frac{A_{tot} \, \varepsilon_M}{}$, $K_{UF}$ ultrafiltration coefficient of the hollow fibers present in the hemodialyzer, $A_{tot}$ total membrane area of the hollow fibers present in the hemodialyzer ($A_{tot} = N\pi d_B L$), $\varepsilon_M$ membrane porosity of the hollow fibers present in the hemodialyzer; |
|---|---|
| $P_{B,out}$ | blood pressure at hemodialyzer exit; |
| $P_{D,out}$ | dialysate pressure at hemodialyzer exit; |
| $\Pi_o$ | average oncotic pressure; |
| $x_i$ | location of inversion point ($J_v(x_i) = 0$); |
| $\mu_B$ | blood viscosity; |
| $Q_B$ | blood flow rate; |
| L | effective length of hollow fibers present in the hemodialyzer; |
| $v_1$ | $J_v(0)$; |
| $v_2$ | $J_v(L)$; |
| $\mu_D$ | dialysate viscosity; |
| $Q_D$ | dialysate flow rate; |
| $f_{R_o,R_k}$ | $\frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}(R_k^2 - R_o^2),$ |

$$R_o = \frac{d_B}{2} + \delta_M,$$

$$R_k = R_o + \delta_\varepsilon,$$

$d_B$ internal diameter of hollow fibers present in the hemodialyzer,
$\delta_M$ wall thickness of the hollow fibers present in the hemodialyzer,
$\delta_\varepsilon$ half the distance between adjacent hollow fibers present in the hemodialyzer.

In a further embodiment of the method, some of the parameters in the above equation for IFR are calculated according to the following equations:

1.

$$f_{R_o,R_k} = \frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}(R_k^2 - R_o^2)$$

2.

$$K = -\frac{K_{UF}}{A_{tot}\,\varepsilon_M}$$

3.

$$E_1 = \frac{\mu_D d_B L^3}{16 f_{R_o,R_k}} - \frac{80\mu_B L^3}{3 d_B^3}$$

4.

$$E_2 = \frac{5\mu_D d_B L^3}{48 f_{R_o,R_k}} - \frac{16\mu_B L^3}{d_B^3}$$

5.

$$R_1 = 1 + \frac{128}{3} K \frac{\mu_B L^2}{d_B^3}$$

6.

$$R_2 = 1 - K \frac{\mu_D d_B L^2}{6 f_{R_o, R_k}} + \frac{16}{9} K^2 \frac{\mu_B \mu_D L^4}{R_1 f_{R_o, R_k} d_B^2}$$

7.

$$F_1 = \left( P_{B,out} - \pi_0 \right) L + 64 \frac{\mu_B L^2 Q_B}{N \pi d_B^4} + \frac{\mu_D L^2 Q_D}{4 N \pi f_{R_o, R_k}}$$

8.

$$F_2 = E_2 - \frac{64}{3} \frac{E_1 \mu_B L^2 K}{R_1 d_B^3}$$

9.

$$B_1 = P_{B,out} - P_{D,out} - \pi_o + \frac{128 \mu_B Q_B L}{N \pi d_B^4}$$

10.

$$B_2 = P_{B,out} - \pi_o + \frac{128 \mu_B Q_B L}{N \pi d_B^4}$$

11.

$$D_1 = P_{B,out} - P_{D,out} - \pi_o + \frac{\mu_D Q_D L}{2 N \pi f_{R_o, R_k}}$$

12.

$$D_2 = P_{B,out} - \pi_o + \frac{\mu_D Q_D L}{2 N \pi f_{R_o, R_k}}$$

13.

$$G_1 = F_1 + \frac{E_1}{R_1} K B_2 + \frac{F_2}{R_2} K D_2 + \frac{F_2}{R_2} K^2 \frac{\mu_D d_B L^2}{12 f_{R_o, R_k}} B_2$$

14.

$$G_2 = L + \frac{E_1}{R_1} K + \frac{F_2}{R_2} K + \frac{F_2}{R_2} K^2 \frac{\mu_D d_B L^2}{12 R_1 f_{R_o, R_k}}$$

15.

$$P_{D,out} = \frac{1}{G_2}\left(G_1 - \frac{UF}{N\pi d_B K}\right)$$

16.

$$v_1 = J_v(0) = K\frac{1}{R_1}\left(B_1 - \frac{64\mu_B L^2}{3d_B^3}v_2\right)$$

17.

$$v_2 = J_v(L) = \frac{K}{R_2}\left(D_1 + K\frac{\mu_D d_B L^2 B_1}{12R_1 f_{R_O,R_k}}\right)$$

18.

$$x_i = \frac{v_1}{v_1 - v_2}$$

with

N        number of hollow fibers present in the hemodialyzer;
L        effective length of the hollow fibers present in the hemodialyzer;
$d_B$       internal diameter of the hollow fibers present in the hemodialyzer;
$A_{tot}$      total membrane area of the hollow fibers present in the hemodialyzer ($A_{tot} = N\pi d_B L$);
UF       net ultrafiltration flow rate;
$K_{UF}$      UF coefficient;
$Q_B$       blood flow rate;
$Q_D$       dialysate flow rate;
$P_{B,out}$     blood pressure at dialyzer exit;
$P_{D,out}$     dialysate pressure at dialyzer exit;
$\Pi_o$       average oncotic pressure;
$\mu_B$       blood viscosity;
$\mu_D$       dialysate viscosity;
$\varepsilon_M$       membrane porosity;
$\delta_M$       membrane thickness.

In one embodiment of the method, the total volume $V_{tot}$ of fluid exchanged through the wall of the hollow fiber membranes present in the hemodialyzer over a time period TD of operation of the hemodialyzer is determined according to

$$V_{tot} = \int_0^{TD} IFR(t)dt$$

The present disclosure also provides a system comprising

a) a database comprising data on physical properties of a plurality of capillary hemodialyzers and of hollow fibers present therein; and/or
b) an input device configured for providing data on physical properties of a capillary hemodialyzer and of hollow fibers present in the hemodialyzer and/or for providing a blood flow rate and a dialysis flow rate through the hemodialyzer during operation of the hemodialyzer;
c) an output device configured for output of data received from a computer processor in operative association with the output device;

d) a computer processor programmed for communication with the database and/or the input device, and for communication with the output device, the processor programmed for

a. acquiring data from the database and/or the input device,

b. determining an internal flow rate IFR within a capillary hemodialyzer, based on the acquired data,

c. optionally, determining a total volume $V_{tot}$ of fluid exchanged through the wall of the hollow fiber membranes present in the hemodialyzer during operation of the hemodialyzer;

d. transmitting the determined internal flow rate IFR, and, optionally, the total volume $V_{tot}$ of fluid exchanged to the output device.

In one embodiment of the system, the input device, the output device, and the processor are contained in a portable device. In one embodiment, the portable device is a portable computer, for instance, a laptop, a tablet computer, or a PDA. In a further embodiment, the portable device is a mobile communication device, for instance, a smartphone.

In one embodiment of the system, the output device is a display device. Examples of suitable display devices include monitors, computer displays, and touchscreens. In a particular embodiment, the display device is a touchscreen of a smartphone.

In one embodiment of the system, the database is present in a computer memory in operative association with the computer processor. In a further embodiment, the computer memory is contained in a portable device comprising the input device, the output device, and the processor. In another embodiment, the database is present in a remote computer memory, a network drive, or a cloud memory accessible via the internet.

The present disclosure also provides a computer program which, when executed on a data processing system, instructs a computer processor to perform the method of

a. acquiring data from a database and/or an input device in operative association with the processor;

b. determining an internal filtration rate IFR within a capillary hemodialyzer, based on the acquired data;

c. optionally, determining a total volume $V_{tot}$ of fluid exchanged through the wall of the hollow fiber membranes present in the hemodialyzer during operation of the hemodialyzer;

d. transmitting the determined internal flow rate IFR, and, optionally, the total volume $V_{tot}$ of fluid exchanged to an output device in operative association with the processor.

In one embodiment, the computer program takes the form of a software application ("app") that can be installed and run on a smartphone.

The present disclosure also provides a non-transitory computer-readable medium comprising the computer program.

It will be understood that the features mentioned above and those described hereinafter can be used not only in the combination specified but also in other combinations or on their own, without departing from the scope of the present invention.

The method of the present disclosure will now be further described in the following examples and referring to the attached drawings.

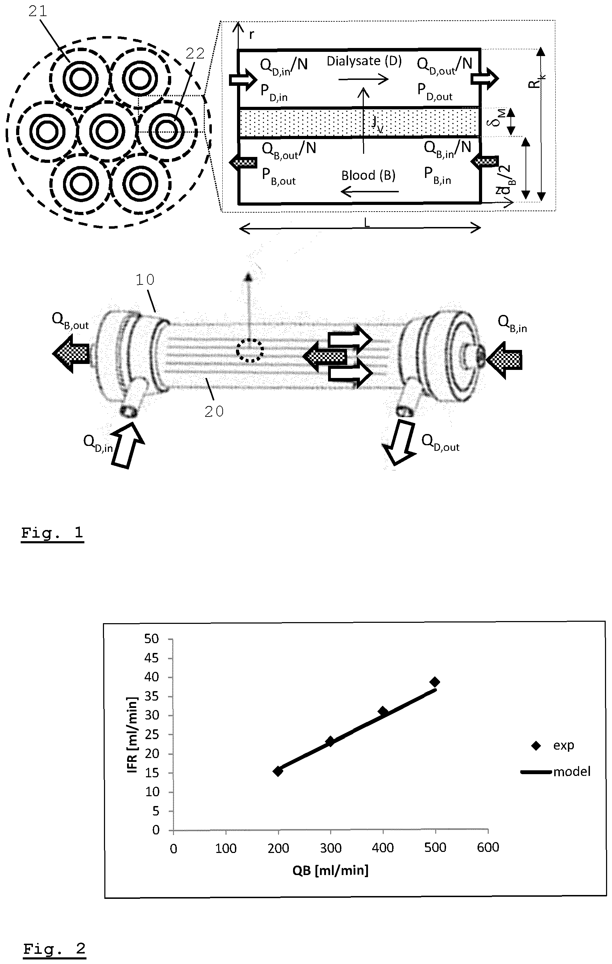

Figure 1 shows a schematic perspective view and sectional detail views of a capillary hemodialyzer 10 and fluid flows within the hemodialyzer 10. The hemodialyzer 10 comprises a bundle 20 of hollow fiber membranes 21. In the top left corner of Fig. 1, a detail of a cross-section of the bundle 20 is shown with several hollow fiber membranes 21, each having a membrane wall 22. Blood flow and dialysate flow through the hemodialyzer 10 are indicated by arrows. Blood enters the hemodialyzer 10 through a blood inlet located on a header of the hemodialyzer 10 at a blood flow rate $Q_{B,in}$, flows through the lumen of the hollow fiber membranes 21 of the bundle 20, and leaves the hemodialyzer 10 at a blood flow rate $Q_{B,out}$ through a blood outlet located on another header of the hemodialyzer 10. Dialysate enters the hemodialyzer 10 through a dialysate inlet positioned near one end of the hemodialyzer 10 at a dialysate flow rate $Q_{D,in}$, flows through the space outside the hollow fiber membranes 21 of the bundle 20, and leaves the hemodialyzer 10 at a dialysate flow rate $Q_{D,out}$ through a dialysate outlet positioned near the opposite end of the hemodialyzer 10. In the top right corner of Fig. 1, a detail of a longitudinal section of one of hollow fiber membranes 21 is shown. The effective length of the hollow fiber membrane 21 is L, the internal radius of the hollow fiber membrane 21 is $d_B/2$, the thickness of the membrane wall 22 is $\delta_M$, the distance between the longitudinal axis of one hollow fiber membrane 21 and the longitudinal axis of an adjacent hollow fiber membrane 21 is $2*R_k$, with $R_k=R_o+\delta_\varepsilon$, or $R_k=d_B/2+\delta_M+\delta_\varepsilon$, $R_o$ being half the outside diameter of the hollow fiber membrane 21, and $\delta_\varepsilon$ being half the distance between two adjacent hollow fiber membranes 21. Blood flows in the lumen of the hollow fiber membrane 21, dialysate flows on the outside of the hollow fiber membrane 21, and ultrafiltration flux $J_v$ permeates the membrane wall 22.

Example 1

Internal filtration rates within a hemodialyzer (Polyflux® 210H, Gambro Dialysatoren GmbH, 72379 Hechingen, Germany) were determined according to one embodiment of the method of the present disclosure, and compared to internal flow rates determined experimentally (from D. Schneditz et al.: "Internal filtration, filtration fraction, and blood flow resistance in high- and low-flux dialyzers", Clin. Hemorheol. Microcirc. 58 (2014) 455-469).

The following physical properties of the hemodialyzer were used for the determination:

- hollow fiber inner diameter $d_B$ = 215 μm
- Membrane wall thickness $\delta_M$ = 50 μm
- Membrane porosity $\varepsilon_M$ = 0.7
- Number of fibers N = 11,640
- Effective length L = 270 mm
- Ultrafiltration coefficient $K_{UF}$ = 85 ml/ (h*mm Hg)
- Half distance between adjacent fibers $\delta_\varepsilon$ = 68.2 μm
- Hemodialyzer housing inner diameter $d_H$ = 48.7 mm

Values for blood viscosity of $\mu_B$ = 5.2 mPas and dialysate viscosity of $\mu_D$ = 0.96 mPas were used.

Internal filtration rate IFR was determined for a dialysis flow rate $Q_D$ of 500 ml/min, and blood flow rates $Q_B$ of 200 ml/min, 300 ml/min, 400 ml/min, and 500 ml/min, respectively.

The values for the following constant parameters were obtained as described above.

- K = 0.119 μm²s/kg

- $R_1$ = 1.19

- $R_2$ = 1.01

- $E_1$ = -285 MPas

- $E_2$ = -182 MPas

- $F_2$ = -159 MPas

- $G_2$ = 0.223 m

The following table shows IFR at $Q_D$ = 500 ml/min and different blood flow rates $Q_B$.

| $Q_B$ [ml/min] | 200 | 300 | 400 | 500 |
|---|---|---|---|---|
| IFR [ml/min] | 15.4 | 23.1 | 30.7 | 38.4 |

Figure 2 shows a comparison of the internal flow rates determined according to the method of the present disclosure and experimentally determined internal flow rates taken from D. Schneditz et al.: "Internal filtration, filtration fraction, and blood flow resistance in high- and low-flux dialyzers", Clin. Hemorheol. Microcirc. 58 (2014) 455-469.

**Example 2**

Internal filtration rates within a hemodialyzer (Theranova® 400, Gambro Dialysatoren GmbH, 72379 Hechingen, Germany) were determined according to one embodiment of the method of the present disclosure, and compared to internal flow rates determined experimentally (from A. Lorenzin et al.: "Quantification of Internal Filtration in Hollow Fiber Hemodialyzers with Medium Cut-off Membrane", Blood Purif. 46 (2018) 196-204).

The following physical properties of the hemodialyzer were used for the determination:

- hollow fiber inner diameter $d_B$ = 180 μm
- Membrane wall thickness $\delta_M$ = 35 μm
- Membrane porosity $\varepsilon_M$ = 0.5

- Number of fibers N = 13,000
- Effective length L = 236 mm
- Ultrafiltration coefficient $K_{UF}$ = 48 ml/ (h*mm Hg)
- half distance between adjacent fibers $\delta_\varepsilon$ = 41.9 $\mu$m
- Hemodialyzer housing inner diameter $d_H$ = 38 mm

Values for blood viscosity of $\mu_B$ = 5.0 mPas and dialysate viscosity of $\mu_D$ = 0.96 mPas were used.

Internal filtration rate IFR was determined for a dialysis flow rate $Q_D$ of 500 ml/min, and blood flow rates $Q_B$ of 300 ml/min and 400 ml/min, respectively.

The values for the following constant parameters were obtained as described above.

- K = 0.116 $\mu$m$^2$s/kg

- $R_1$ = 1.24

- $R_2$ = 1.04

- $E_1$ = -335 MPas

- $E_2$ = -237 MPas

- $F_2$ = -206 MPas

- $G_2$ = 0.182 m

The following table shows IFR at $Q_D$ = 500 ml/min and different blood flow rates $Q_B$.

| $Q_B$ [ml/min] | 300 | 400 |
|---|---|---|
| IFR [ml/min] | 26.7 | 41.6 |

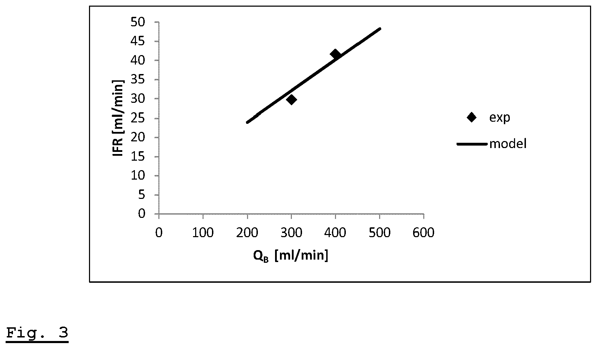

Figure 3 shows a comparison of the internal flow rates determined according to the method of the present disclosure and experimentally determined internal flow rates taken from A. Lorenzin et al.: "Quantification of Internal Filtration in Hollow Fiber Hemodialyzers with Medium Cut-off Membrane", Blood Purif. 46 (2018) 196-204.

List of reference signs

10      hemodialyzer
20      bundle of hollow fiber membranes
21      hollow fiber membrane
22      membrane wall
L       length of hollow fiber membrane
$\delta_M$      thickness of membrane wall
$d_B$      internal diameter of hollow fiber membrane
$R_k$      radius of space occupied by hollow fiber membrane

Claims

1.  A computer-implemented method for determining an internal filtration rate IFR within a capillary hemodialyzer (10) using the dimensions of the hemodialyzer, dimensions and physical parameters of the hollow fiber membranes present in the hemodialyzer, and flow rates of blood and dialysate through the hemodialyzer, the method comprising

    i) acquiring, using a computer, data on physical properties of the hemodialyzer (10) and of hollow fiber membranes (21) present in the hemodialyzer (10), the data comprising a diameter $d_H$ of the housing of the hemodialyzer (10), a number N of the hollow fiber membranes (21) present in the hemodialyzer (10), an effective length L of the hollow fiber membranes (21) present in the hemodialyzer (10), a total surface area $A_{tot}$ of the hollow fiber membranes (21) present in the hemodialyzer (10), an internal diameter $d_B$ of the hollow fiber membranes (21) present in the hemodialyzer (10), a wall thickness $\delta_M$ of the hollow fiber membranes (21) present in the hemodialyzer (10), a porosity $\varepsilon_M$ of the hollow fiber membranes (21) present in the hemodialyzer (10), and an ultrafiltration coefficient $K_{UF}$ of the hollow fiber membranes (21) present in the hemodialyzer (10);

ii) acquiring, using the computer, a previously stored blood flow rate $Q_B$ and a dialysis flow rate $Q_D$ through the hemodialyzer (10) relating to a past operation of the hemodialyzer (10);

iii) determining, using the computer, the internal filtration rate IFR of the hemodialyzer (10), based on the data acquired in steps i and ii, wherein the internal filtration rate IFR is determined according to

$$
\begin{aligned}
IFR = N\pi d_B\, K \Bigg\{ & \left(P_{B,out} - P_{D,out} - \pi_0\right)x_i \\
& -\frac{128\mu_B}{N\pi d_B^4}\left[Q_B\left(\frac{x_i^2}{2} - Lx_i\right)\right. \\
& \left. - N\pi d_B\left[\frac{v_2 - v_1}{6L}\left(\frac{x_i^4}{4} - L^3 x_i\right) + \frac{v_1}{2}\left(\frac{x_i^3}{3} - L^2 x_i\right)\right]\right] \\
& + \frac{\mu_D}{2N\pi f_{R_o,R_k}}\left[Q_D\,\frac{x_i^2}{2} - N\pi d_B\left[\frac{v_2 - v_1}{2L}\left(\frac{x_i^4}{12} - \frac{L^2}{2}x_i^2\right) + v_1\left(\frac{x_i^3}{6} - \frac{L}{2}x_i^2\right)\right]\right]\Bigg\}
\end{aligned}
$$

with

N number of hollow fiber membranes (21) present in the hemodialyzer (10);

$d_B$ internal diameter of hollow fiber membranes (21) present in the hemodialyzer (10);

K $\dfrac{K_{UF}}{A_{tot}\,\varepsilon_M}$ ,

$K_{UF}$ ultrafiltration coefficient of the hollow fiber membranes (21) present in the hemodialyzer (10),

$A_{tot}$ total membrane area of the hollow fiber membranes (21) present in the hemodialyzer (10) ($A_{tot} = N\pi d_B L$),

$\varepsilon_M$ membrane porosity of the hollow fiber membranes (21) present in the hemodialyzer (10);

$P_{B,out}$ blood pressure at hemodialyzer exit;

$P_{D,out}$ dialysate pressure at hemodialyzer exit;

$\Pi_0$ average oncotic pressure;

$x_i$ location of inversion point ($J_v(x_i) = 0$);

$\mu_B$ blood viscosity;

$Q_B$ blood flow rate;

L effective length of hollow fiber membranes (21) present in the hemodialyzer (10);

$v_1$ $J_v(0)$;

$v_2$ $J_v(L)$;

$\mu_D$ dialysate viscosity;

$Q_D$ dialysate flow rate;

$f_{R_o,R_k}$

$$
\frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right),
$$

$$R_o = \frac{d_B}{2} + \delta_M,$$

$$R_k = R_o + \delta_\varepsilon,$$

$d_B$ internal diameter of the hollow fiber membranes (21) present in the hemodialyzer (10),
$\delta_M$ wall thickness of the hollow fiber membranes (21) present in the hemodialyzer (10),
$\delta_\varepsilon$ thickness of the diffusion layer around the hollow fiber membranes (21) present in the hemodialyzer (10).

2. The method of claim 1, additionally comprising

iv) acquiring, using the computer, data on a duration TD of the operation of the hemodialyzer (10) ;
v) determining, using the computer, a total volume $V_{tot}$ of fluid exchanged through the wall (22) of the hollow fiber membranes (21) present in the hemodialyzer (10) during operation of the hemodialyzer (10), based on the internal filtration rate IFR and the data acquired in step iv, wherein the total volume $V_{tot}$ is determined according to

$$V_{tot} = \int_0^{TD} IFR(t)dt$$

3. The method of claim 1 or 2, wherein data on physical properties of the hemodialyzer (10) and of hollow fiber membranes (21) present in the hemodialyzer (10) are acquired from a database in operative association with a processor of the computer.

4. The method of any one of claims 1 to 3, wherein data on physical properties of the hemodialyzer (10) and of hollow fiber membranes (21) present in the hemodialyzer (10) and/or the blood flow rate and the dialysis flow rate through the hemodialyzer (10) during operation of the hemodialyzer (10) is acquired from an input device in operative association with a processor of the computer.

5. The method of claim 4, wherein the input device comprises a contactless reader.

6. The method of claim 4 or 5, wherein the input device comprises at least one user interface.

7. The method of any one of claims 4 to 6, wherein the input device comprises an extracorporeal blood treatment apparatus.

8. A system comprising

a) a database comprising data on physical properties of a plurality of capillary hemodialyzers (10) and of hollow fiber membranes (21) present therein, the data comprising a diameter $d_H$ of the housing of the hemodialyzers (10), a number N of the hollow fiber membranes (21) present in the hemodialyzers (10), an effective length L of the hollow fiber membranes (21) present in the hemodialyzers (10), a total surface area $A_{tot}$ of the hollow fiber membranes (21) present in the hemodialyzers (10), an internal diameter $d_B$ of the hollow fiber membranes (21) present in the hemodialyzers (10), a wall thickness $\delta_M$ of the hollow fiber membranes (21) present in the hemodialyzers (10), a porosity $\varepsilon_M$ of the hollow fiber membranes (21) present in the hemodialyzers (10), and an ultrafiltration coefficient $K_{UF}$ of the hollow fiber membranes (21) present in the hemodialyzers (10); and/or
b) an input device configured for providing data on physical properties of a capillary hemodialyzer (10) and of hollow fiber membranes (21) present in the hemodialyzer (10), the data comprising a diameter $d_H$ of the housing of the hemodialyzer (10), a number N of the hollow fiber membranes (21) present in the hemodialyzer (10), an effective length L of the hollow fiber membranes (21) present in the hemodialyzer (10), a total surface area $A_{tot}$ of the hollow fiber membranes (21) present in the hemodialyzer (10), an internal diameter $d_B$ of the hollow fiber membranes (21) present in the hemodialyzer (10), a wall thickness $\delta_M$ of the hollow fiber membranes (21) present in the hemodialyzer (10), a porosity $\varepsilon_M$ of the hollow fiber membranes (21) present in the hemodialyzer (10), and an ultrafiltration coefficient $K_{UF}$ of the hollow fiber membranes (21) present in the hemodialyzer (10);

and/or for providing a blood flow rate and a dialysis flow rate through the hemodialyzer (10) during operation of the hemodialyzer (10);

c) an output device configured for output of data received from a computer processor in operative association with the output device;

d) a computer processor programmed for communication with the database and/or the input device, and for communication with the output device, the processor programmed for

a. acquiring data from the database and/or the input device, the data comprising a diameter $d_H$ of the housing of a hemodialyzer (10), a number N of the hollow fiber membranes (21) present in the hemodialyzer (10), an effective length L of the hollow fiber membranes (21) present in the hemodialyzer (10), a total surface area $A_{tot}$ of the hollow fiber membranes (21) present in the hemodialyzer (10), an internal diameter $d_B$ of the hollow fiber membranes (21) present in the hemodialyzer (10), a wall thickness $\delta_M$ of the hollow fiber membranes (21) present in the hemodialyzer (10), a porosity $\varepsilon_M$ of the hollow fiber membranes (21) present in the hemodialyzer (10), and an ultrafiltration coefficient $K_{UF}$ of the hollow fiber membranes (21) present in the hemodialyzer (10); and/or a blood flow rate and a dialysis flow rate through the hemodialyzer (10) during operation of the hemodialyzer (10),

b. determining an internal filtration rate IFR within a capillary hemodialyzer (10), based on the acquired data, wherein the internal filtration rate IFR is determined according to

$$
\begin{aligned}
IFR = N\pi d_B \, K \Bigg\{ & \left(P_{B,out} - P_{D,out} - \pi_0\right)x_i \\
& - \frac{128\mu_B}{N\pi d_B^4}\left[Q_B\left(\frac{x_i^2}{2} - Lx_i\right)\right. \\
& \left. - N\pi d_B\left[\frac{\nu_2 - \nu_1}{6L}\left(\frac{x_i^4}{4} - L^3 x_i\right) + \frac{\nu_1}{2}\left(\frac{x_i^3}{3} - L^2 x_i\right)\right]\right] \\
& + \frac{\mu_D}{2N\pi f_{R_o,R_k}}\left[Q_D\frac{x_i^2}{2} - N\pi d_B\left[\frac{\nu_2 - \nu_1}{2L}\left(\frac{x_i^4}{12} - \frac{L^2}{2}x_i^2\right) + \nu_1\left(\frac{x_i^3}{6} - \frac{L}{2}x_i^2\right)\right]\right]\Bigg\}
\end{aligned}
$$

with

N number of hollow fiber membranes (21) present in the hemodialyzer (10);

$d_B$ internal diameter of hollow fiber membranes (21) present in the hemodialyzer (10) ;

K $\dfrac{K_{UF}}{A_{tot}\,\varepsilon_M}$ ,

$K_{UF}$ ultrafiltration coefficient of the hollow fiber membranes (21) present in the hemodialyzer (10),

$A_{tot}$ total membrane area of the hollow fiber membranes (21) present in the hemodialyzer (10) ($A_{tot} = N\pi d_B L$),

$\varepsilon_M$ membrane porosity of the hollow fiber membranes (21) present in the hemodialyzer (10) ;

$P_{B,out}$ blood pressure at hemodialyzer exit;

$P_{D,out}$ dialysate pressure at hemodialyzer exit;

$\Pi_0$ average oncotic pressure;

$x_i$ location of inversion point ($J_v(x_i) = 0$);

$\mu_B$ blood viscosity;

$Q_B$ blood flow rate;

L effective length of hollow fiber membranes (21) present in the hemodialyzer (10);

$\nu_1$ $J_v(0)$;

$\nu_2$ $J_v(L)$;

$\mu_D$ dialysate viscosity;

$Q_D$ dialysate flow rate;

$f_{R_o,R_k}$

$$\frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right),$$

$$R_o = \frac{d_B}{2} + \delta_M,$$

$$R_k = R_o + \delta_\varepsilon,$$

$d_B$ internal diameter of the hollow fiber membranes (21) present in the hemodialyzer (10),
$\delta_M$ wall thickness of the hollow fiber membranes (21) present in the hemodialyzer (10),
$\delta_\varepsilon$ thickness of the diffusion layer around the hollow fiber membranes (21) present in the hemodialyzer (10),

c. optionally, determining a total volume $V_{tot}$ of fluid exchanged through the wall of the hollow fiber membranes (21) present in the hemodialyzer (10) during operation of the hemodialyzer (10), wherein the total volume $V_{tot}$ is determined according to

$$V_{tot} = \int_0^{TD} IFR(t)dt\,;$$

d. transmitting the determined internal filtration rate IFR, and, optionally, the total volume $V_{tot}$ of fluid exchanged to the output device.

9. The system of claim 8, wherein the input device, the output device, and the processor are contained in a mobile communication device.

10. The system of claim 8 or 9, wherein the input device is a graphical use interface (GUI) of a mobile communication device.

11. The system of any one of claims 8 to 10, wherein the output device is a display device.

12. A computer program which, when executed on a data processing system, instructs a computer processor to perform the method of

a. acquiring data from a database and/or an input device in operative association with the processor, the data comprising a diameter $d_H$ of the housing of a hemodialyzer (10), a number N of the hollow fiber membranes (21) present in the hemodialyzer (10), an effective length L of the hollow fiber membranes (21) present in the hemodialyzer (10), a total surface area $A_{tot}$ of the hollow fiber membranes (21) present in the hemodialyzer (10), an internal diameter $d_B$ of the hollow fiber membranes (21) present in the hemodialyzer (10), a wall thickness $\delta_M$ of the hollow fiber membranes (21) present in the hemodialyzer (10), a porosity $\varepsilon_M$ of the hollow fiber membranes (21) present in the hemodialyzer (10), and an ultrafiltration coefficient $K_{UF}$ of the hollow fiber membranes (21) present in the hemodialyzer (10); and/or a blood flow rate and a dialysis flow rate through the hemodialyzer (10) during operation of the hemodialyzer (10);
b. determining an internal filtration rate IFR within a capillary hemodialyzer (10), based on the acquired data, wherein the internal filtration rate IFR is determined according to

$$IFR = N\pi d_B\, K \left\{ (P_{B,out} - P_{D,out} - \pi_0)x_i \right.$$

$$- \frac{128\mu_B}{N\pi d_B^4}\left[ Q_B\left( \frac{x_i^2}{2} - Lx_i \right) \right.$$

$$\left. - N\pi d_B\left[ \frac{v_2 - v_1}{6L}\left( \frac{x_i^4}{4} - L^3 x_i \right) + \frac{v_1}{2}\left( \frac{x_i^3}{3} - L^2 x_i \right) \right] \right]$$

$$\left. + \frac{\mu_D}{2N\pi f_{R_o,R_k}}\left[ Q_D\,\frac{x_i^2}{2} - N\pi d_B\left[ \frac{v_2 - v_1}{2L}\left( \frac{x_i^4}{12} - \frac{L^2}{2}x_i^2 \right) + v_1\left( \frac{x_i^3}{6} - \frac{L}{2}x_i^2 \right) \right] \right] \right\}$$

with

N number of hollow fiber membranes (21) present in the hemodialyzer (10);
$d_B$ internal diameter of hollow fiber membranes (21) present in the hemodialyzer (10) ;
$$K \quad \frac{K_{UF}}{A_{tot}\,\varepsilon_M}\;,$$
$K_{UF}$ ultrafiltration coefficient of the hollow fiber membranes (21) present in the hemodialyzer (10),
$A_{tot}$ total membrane area of the hollow fiber membranes (21) present in the hemodialyzer (10) ($A_{tot} = N\pi d_B L$),
$\varepsilon_M$ membrane porosity of the hollow fiber membranes (21) present in the hemodialyzer (10) ;
$P_{B,out}$ blood pressure at hemodialyzer exit;
$P_{D,out}$ dialysate pressure at hemodialyzer exit;
$\Pi_0$ average oncotic pressure;
$x_i$ location of inversion point ($J_v(x_i) = 0$) ;
$\mu_B$ blood viscosity;
$Q_B$ blood flow rate;
L effective length of hollow fiber membranes (21) present in the hemodialyzer (10);
$v_1$ $J_v(0)$;
$v_2$ $J_v(L)$;
$\mu_D$ dialysate viscosity;
$Q_D$ dialysate flow rate;
$f_{R_o,R_k}$

$$\frac{R_k^4 - R_o^4}{16} + \frac{R_k^2}{2}\left( \frac{R_k^2}{2}\ln\left( \frac{R_o}{R_k} \right) + \frac{R_k^2 - R_o^2}{4} \right) - \frac{R_o^2}{8}(R_k^2 - R_o^2),$$

$$R_o = \frac{d_B}{2} + \delta_M,$$

$$R_k = R_o + \delta_\varepsilon,$$

$d_B$ internal diameter of the hollow fiber membranes (21) present in the hemodialyzer (10),
$\delta_M$ wall thickness of the hollow fiber membranes (21) present in the hemodialyzer (10),
$\delta_\varepsilon$ thickness of the diffusion layer around the hollow fiber membranes (21) present in the hemodialyzer (10);

c. optionally, determining a total volume $V_{tot}$ of fluid exchanged through the wall of the hollow fiber membranes (21) present in the hemodialyzer (10) during operation of the hemodialyzer (10), wherein the total volume $V_{tot}$ is determined according to

$$V_{tot} = \int_0^{TD} IFR(t)dt \, ;$$

d. transmitting the determined internal filtration rate IFR, and, optionally, the total volume $V_{tot}$ of fluid exchanged to an output device in operative association with the processor.

**Patentansprüche**

1. Computerimplementiertes Verfahren zur Bestimmung einer internen Filtrationsrate IFR innerhalb eines Kapillarhämodialysators (10) unter Verwendung von Abmessungen des Hämodialysators, von Abmessungen und physikalischen Parametern der Hohlfasermembranen, die im Hämodialysator vorhanden sind, und von Flussraten von Blut und Dialysat durch den Hämodialysator, wobei das Verfahren umfasst:

i) die Erfassung von Daten über physikalische Eigenschaften des Hämodialysators (10) und von im Hämodialysator (10) vorhandenen Hohlfasermembranen (21) unter Verwendung eines Computers, wobei die Daten einen Durchmesser $d_H$ des Gehäuses des Hämodialysators (10), eine Zahl N der in dem Hämodialysator (10) vorhandenen Hohlfasermembranen (21), eine effektive Länge L der in dem Hämodialysator (10) vorhandenen Hohlfasermembranen (21), eine Gesamtoberfläche $A_{tot}$ der in dem Hämodialysator (10) vorhandenen Hohlfasermembranen (21), einen Innendurchmesser $d_B$ der in dem Hämodialysator (10) vorhandenen Hohlfasermembranen (21), eine Wandstärke $\delta_M$ der in dem Hämodialysator (10) vorhandenen Hohlfasermembranen (21), eine Porosität $\varepsilon_M$ der in dem Hämodialysator (10) vorhandenen Hohlfasermembranen (21), und einen Ultrafiltrationskoeffizienten $K_{UF}$ der in dem Hämodialysator (10) vorhandenen Hohlfasermembranen (21) umfassen;
ii) die Erfassung einer zuvor gespeicherten Blutflussrate $Q_B$ und einer Dialysatflussrate $Q_D$ durch den Hämodialysator (10), die sich auf einen früheren Betrieb des Hämodialysators (10) beziehen, unter Verwendung des Computers;
iii) die Bestimmung der internen Filtrationsrate IFR des Hämodialysators (10) unter Verwendung des Computers auf der Grundlage der in den Schritten i und ii erfassten Daten, wobei die interne Filtrationsrate IFR bestimmt wird gemäß

$$
\begin{aligned}
IFR = N\pi d_B K \Bigg\{ & \left(P_{B,out} - P_{D,out} - \pi_0\right)x_i \\
& - \frac{128\mu_B}{N\pi d_B^4}\left[Q_B\left(\frac{x_i^2}{2} - Lx_i\right)\right. \\
& \left. - N\pi d_B\left[\frac{v_2 - v_1}{6L}\left(\frac{x_i^4}{4} - L^3 x_i\right) + \frac{v_1}{2}\left(\frac{x_i^3}{3} - L^2 x_i\right)\right]\right] \\
& + \frac{\mu_D}{2N\pi f_{R_o,R_k}}\left[Q_D\frac{x_i^2}{2} - N\pi d_B\left[\frac{v_2 - v_1}{2L}\left(\frac{x_i^4}{12} - \frac{L^2}{2}x_i^2\right) + v_1\left(\frac{x_i^3}{6} - \frac{L}{2}x_i^2\right)\right]\right] \Bigg\}
\end{aligned}
$$

mit

N Anzahl der im Hämodialysator (10) vorhandenen Hohlfasermembranen (21);
$d_B$ Innendurchmesser der im Hämodialysator (10) vorhandenen Hohlfasermembranen (21);

K, $\dfrac{K_{UF}}{A_{tot}\,\varepsilon_M}$

$K_{UF}$ Ultrafiltrationskoeffizient der im Hämodialysator (10) vorhandenen Hohlfasermembranen (21),
$A_{tot}$ Gesamtmembranfläche der in dem Hämodialysator (10) vorhanden Hohlfasermembranen (21),
$(A_{tot} = N\pi d_B L)$,
$\varepsilon_M$ Membranporosität der in dem Hämodialysator (10) vorhandenen Hohlfasermembranen (21);

$P_{B,out}$ Blutdruck am Ausgang des Hämodialysators;

$P_{D,out}$ Dialysatdruck am Ausgang des Hämodialysators;

$\Pi_0$ mittlerer onkotischer Druck;

$x_i$ Lage des Inversionspunktes ($J_v(x_i) = 0$);

$\mu_B$ Blutviskosität;

$Q_B$ Blutflussrate;

L effektive Länge der in dem Hämodialysator (10) vorhandenen Hohlfasermembranen (21);

$v_1$ $J_v(0)$;

$v_2$ $J_v(L)$

$\mu_D$ Viskosität des Dialysats;

$Q_D$ Dialysatflussrate;

$fR_o, R_k$

$$\frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right),$$

$$R_o = \frac{d_B}{2} + \delta_M,$$

$$R_k = R_o + \delta_\varepsilon,$$

$d_B$ Innendurchmesser der im Hämodialysator (10) vorhandenen Hohlfasermembranen (21),

$\delta_M$ Wandstärke der im Hämodialysator (10) vorhandenen Hohlfasermembranen (21),

$\delta_\varepsilon$ Dicke der Diffusionsschicht um die im Hämodialysator (10) vorhandenen Hohlfasermembranen (21).

2. Verfahren nach Anspruch 1, umfassend zusätzlich

iv) die Erfassung von Daten über eine Betriebsdauer TD des Hämodialysators (10) mit Hilfe des Computers;

v) die Bestimmung des Gesamtvolumens $V_{tot}$ der Flüssigkeit, die während des Betriebs des Hämodialysators (10) durch die Wand (22) der im Hämodialysator (10) vorhandenen Hohlfasermembranen (21) ausgetauscht wird, auf der Grundlage der internen Filtrationsrate IFR und der in Schritt iv erfassten Daten unter Verwendung des Computers, wobei das Gesamtvolumen $V_{tot}$ bestimmt wird gemäß

$$V_{tot} = \int_0^{TD} IFR(t)\,dt$$

3. Verfahren nach Anspruch 1 oder 2, worin Daten über physikalische Eigenschaften des Hämodialysators (10) und der im Hämodialysator (10) vorhandenen Hohlfasermembranen (21) aus einer Datenbank in operativer Verbindung mit einem Prozessor des Computers erlangt werden.

4. Verfahren nach einem der Ansprüche 1 bis 3, worin Daten über physikalische Eigenschaften des Hämodialysators (10) und der im Hämodialysator (10) vorhandenen Hohlfasermembranen (21) und/oder der Blutflussrate und der Dialysatflussrate durch den Hämodialysator (10) während des Betriebs des Hämodialysators (10) von einer Eingabevorrichtung in operativer Verbindung mit einem Prozessor des Computers erfasst werden.

5. Verfahren nach Anspruch 4, wobei die Eingabevorrichtung ein berührungsloses Lesegerät umfasst.

6. Verfahren nach Anspruch 4 oder 5, wobei die Eingabevorrichtung mindestens eine Benutzerschnittstelle aufweist.

7. Verfahren nach einem der Ansprüche 4 bis 6, worin die Eingabevorrichtung eine extrakorporale Blutbehandlungsvorrichtung umfasst.

8. System, umfassend:

a) eine Datenbank, die Daten über physikalische Eigenschaften einer Vielzahl von Kapillarhämodialysatoren (10) und von darin vorhandenen Hohlfasermembranen (21) umfasst, wobei die Daten einen Durchmesser $d_H$ des Gehäuses der Hämodialysatoren (10), eine Anzahl N der in den Hämodialysatoren (10) vorhandenen Hohlfasermembranen (21), eine effektive Länge L der in den Hämodialysatoren (10) vorhandenen Hohlfasermembranen (21), eine Gesamtfläche $A_{tot}$ der in den Hämodialysatoren (10) vorhandenen Hohlfasermembranen (21), einen Innendurchmesser $d_B$ der in den Hämodialysatoren (10) vorhandenen Hohlfasermembranen (21), eine Wandstärke $\delta_M$ der in den Hämodialysatoren (10) vorhandenen Hohlfasermembranen (21), eine Porosität $\varepsilon_M$ der in den Hämodialysatoren (10) vorhandenen Hohlfasermembranen (21) und einen Ultrafiltrationskoeffizienten $K_{uF}$ der in den Hämodialysatoren (10) vorhandenen Hohlfasermembranen (21) umfassen; und/oder

b) eine Eingabevorrichtung, die zur Bereitstellung von Daten über die physikalischen Eigenschaften eines Kapillarhämodialysators (10) und der in dem Hämodialysator (10) vorhandenen Hohlfasermembranen (21) konfiguriert ist, wobei die Daten einen Durchmesser $d_H$ des Gehäuses des Hämodialysators (10), eine Anzahl N der im Hämodialysator (10) vorhandenen Hohlfasermembranen (21), eine effektive Länge L der im Hämodialysator (10) vorhandenen Hohlfasermembranen (21), eine Gesamtoberfläche $A_{tot}$ der im Hämodialysator (10) vorhandenen Hohlfasermembranen (21), einen Innendurchmesser $d_B$ der im Hämodialysator (10) vorhandenen Hohlfasermembranen (21), eine Wandstärke $\delta_M$ der im Hämodialysator (10) vorhandenen Hohlfasermembranen (21), eine Porosität $\varepsilon_M$ der im Hämodialysator (10) vorhandenen Hohlfasermembranen (21), und einen Ultrafiltrationskoeffizienten $K_{uF}$ der in dem Hämodialysator (10) vorhandenen Hohlfasermembranen (21) umfassen; und/oder zur Bereitstellung einer Blutflussrate und einer Dialysatflussrate durch den Hämodialysator (10) während des Betriebs des Hämodialysators (10);

c) eine Ausgabevorrichtung, die für die Ausgabe von Daten konfiguriert ist, die von einem Computerprozessor in operativer Verbindung mit der Ausgabevorrichtung empfangen werden;

d) einen Computerprozessor, der für die Kommunikation mit der Datenbank und/oder dem Eingabegerät programmiert ist, und für die Kommunikation mit dem Ausgabegerät, wobei der Prozessor programmiert ist für

a. das Erfassen von Daten aus der Datenbank und/oder dem Eingabegerät, wobei die Daten einen Durchmesser $d_H$ des Gehäuses eines Hämodialysators (10), eine Anzahl N der in dem Hämodialysator (10) vorhandenen Hohlfasermembranen (21), eine effektive Länge L der im Hämodialysator (10) vorhandenen Hohlfasermembranen (21), eine Gesamtoberfläche $A_{tot}$ der in dem Hämodialysator (10) vorhandenen Hohlfasermembranen (21), einen Innendurchmesser $d_B$ der im Hämodialysator (10) vorhandenen Hohlfasermembranen (21), eine Wandstärke $\delta_M$ der im Hämodialysator (10) vorhandenen Hohlfasermembranen (21), eine Porosität $\varepsilon_M$ der im Hämodialysator (10) vorhandenen Hohlfasermembranen (21) und einen Ultrafiltrationskoeffizienten $K_{uF}$ der in dem Hämodialysator (10) vorhandenen Hohlfasermembranen (21) umfassen; und/oder eine Blutflussrate und eine Dialysatflussrate durch den Hämodialysator (10) während des Betriebs des Hämodialysators (10),

b. die Bestimmung einer internen Filtrationsrate IFR innerhalb eines Kapillarhämodialysators (10), basierend auf den erfassten Daten, wobei die interne Filtrationsrate IFR bestimmt wird gemäß

$$IFR = N\pi d_B K \left\{ \left(P_{B,out} - P_{D,out} - \pi_0\right)x_i \right.$$

$$- \frac{128\mu_B}{N\pi d_B^4}\left[Q_B\left(\frac{x_i^2}{2} - Lx_i\right)\right.$$

$$\left. - N\pi d_B\left[\frac{v_2 - v_1}{6L}\left(\frac{x_i^4}{4} - L^3 x_i\right) + \frac{v_1}{2}\left(\frac{x_i^3}{3} - L^2 x_i\right)\right]\right]$$

$$\left. + \frac{\mu_D}{2N\pi f_{R_o,R_k}}\left[Q_D\frac{x_i^2}{2} - N\pi d_B\left[\frac{v_2 - v_1}{2L}\left(\frac{x_i^4}{12} - \frac{L^2}{2}x_i^2\right) + v_1\left(\frac{x_i^3}{6} - \frac{L}{2}x_i^2\right)\right]\right]\right\}$$

mit

N Anzahl der im Hämodialysator (10) vorhandenen Hohlfasermembranen (21);
$d_B$ Innendurchmesser der im Hämodialysator (10) vorhandenen Hohlfasermembranen (21);

$$K^{\dfrac{K_{UF}}{A_{tot}\,\varepsilon_M}},$$

$K_{UF}$ Ultrafiltrationskoeffizient der im Hämodialysator (10) vorhandenen Hohlfasermembranen (21),
$A_{tot}$ Gesamtmembranfläche der in dem Hämodialysator (10) vorhandenen Hohlfasermembranen (21) ($A_{tot} = N\pi d_B L$),
$\varepsilon_M$ Membranporosität der im Hämodialysator (10) vorhandenen Hohlfasermembranen (21) ;

$P_{B,out}$ Blutdruck am Ausgang des Hämodialysators;
$P_{D,out}$ Dialysatdruck am Ausgang des Hämodialysators;
$\Pi_0$ mittlerer onkotischer Druck;
$x_i$ Lage des Inversionspunktes ($J_v(x_i) = 0$);
$\mu_B$ Blutviskosität;
$Q_B$ Blutflussrate;
L effektive Länge der im Hämodialysator (10) vorhandenen Hohlfasermembranen (21);
$v_1$ $J_v(0)$;
$v_2$ $J_v(L)$;
$\mu_D$ Viskosität des Dialysats;
$Q_D$ Dialysatflussrate;
$f_{R_o,R_k}$

$$\frac{R_k^4-R_o^4}{16} + \frac{R_k^2}{2}\left(\frac{R_k^2}{2}\ln\left(\frac{R_o}{R_k}\right) + \frac{R_k^2-R_o^2}{4}\right) - \frac{R_o^2}{8}\left(R_k^2 - R_o^2\right),$$

$$R_o = \frac{d_B}{2} + \delta_M,$$

$$R_k = R_o + \delta_\varepsilon,$$

$d_B$ Innendurchmesser der im Hämodialysator (10) vorhandenen Hohlfasermembranen (21),
$\delta_M$ Wandstärke der im Hämodialysator (10) vorhandenen Hohlfasermembranen (21),
$\delta_\varepsilon$ Dicke der Diffusionsschicht um die im Hämodialysator (10) vorhandenen Hohlfasermembranen (21),

c. gegebenenfalls die Bestimmung eines Gesamtvolumens $V_{tot}$ von Flüssigkeit, die während des Betriebs des Hämodialysators (10) durch die Wand der im Hämodialysator (10) vorhandenen Hohlfasermembranen (21) ausgetauscht wird, wobei das Gesamtvolumen $V_{tot}$ bestimmt wird gemäß

$$V_{tot} = \int_0^{TD} IFR(t)dt;$$

d. Übertragung der ermittelten internen Filtrationsrate IFR und gegebenenfalls des Gesamtvolumens $V_{tot}$ der ausgetauschten Flüssigkeit an die Ausgabevorrichtung.

9. System nach Anspruch 8, wobei die Eingabevorrichtung, die Ausgabevorrichtung und der Prozessor in einer mobilen Kommunikationsvorrichtung enthalten sind.

10. System nach Anspruch 8 oder 9, wobei die Eingabevorrichtung eine grafische Benutzerschnittstelle (GUI) einer mobilen Kommunikationsvorrichtung ist.

11. System nach einem der Ansprüche 8 bis 10, wobei die Ausgabevorrichtung eine Anzeigevorrichtung ist.

12. Computerprogramm, das, wenn es auf einem Datenverarbeitungssystem ausgeführt wird, einen Computerprozessor anweist, das folgende Verfahren durchzuführen:

a. Erfassen von Daten aus einer Datenbank und/oder einem Eingabegerät in operativer Verbindung mit dem Prozessor, wobei die Daten einen Durchmesser $d_H$ des Gehäuses eines Hämodialysators (10), eine Anzahl N der im Hämodialysator (10) vorhandenen Hohlfasermembranen (21), eine effektive Länge L der im Hämodialysator (10) vorhandenen Hohlfasermembranen (21) umfassen, eine Gesamtoberfläche $A_{tot}$ der in dem Hämodialysator (10) vorhandenen Hohlfasermembranen (21), einen Innendurchmesser $d_B$ der in dem Hämodialysator (10) vorhandenen Hohlfasermembranen (21), eine Wandstärke $\delta_M$ der in dem Hämodialysator (10) vorhandenen Hohlfasermembranen (21), eine Porosität $\varepsilon_M$ der in dem Hämodialysator (10) vorhandenen Hohlfasermembranen (21), und einen Ultrafiltrationskoeffizienten $K_{UF}$ der in dem Hämodialysator (10) vorhandenen Hohlfasermembranen (21) umfassen; und/oder eine Blutflussrate und eine Dialysatflussrate durch den Hämodialysator (10) während des Betriebs des Hämodialysators (10);

b. Bestimmen einer internen Filtrationsrate IFR innerhalb eines Kapillarhämodialysators (10) auf der Grundlage der erfassten Daten, wobei die interne Filtrationsrate IFR bestimmt wird gemäß

$$IFR = N\pi d_B K \left\{ \left(P_{B,out} - P_{D,out} - \pi_0\right)x_i \right.$$

$$- \frac{128\mu_B}{N\pi d_B^4}\left[Q_B\left(\frac{x_i^2}{2} - Lx_i\right)\right.$$

$$- N\pi d_B\left[\frac{\nu_2 - \nu_1}{6L}\left(\frac{x_i^4}{4} - L^3 x_i\right) + \frac{\nu_1}{2}\left(\frac{x_i^3}{3} - L^2 x_i\right)\right]\right]$$

$$\left. + \frac{\mu_D}{2N\pi f_{R_o,R_k}}\left[Q_D\frac{x_i^2}{2} - N\pi d_B\left[\frac{\nu_2 - \nu_1}{2L}\left(\frac{x_i^4}{12} - \frac{L^2}{2}x_i^2\right) + \nu_1\left(\frac{x_i^3}{6} - \frac{L}{2}x_i^2\right)\right]\right]\right\}$$

mit

N Anzahl der im Hämodialysator (10) vorhandenen Hohlfasermembranen (21);

$d_B$ Innendurchmesser der im Hämodialysator (10) vorhanden Hohlfasermembranen (21);

$K = \frac{K_{UF}}{A_{tot}\,\varepsilon_M}$ , $K_{UF}$ Ultrafiltrationskoeffizient der im Hämodialysator (10) vorhandenen Hohlfasermembranen (21), $A_{tot}$ Gesamtmembranfläche der in dem Hämodialysator (10) vorhandenen Hohlfasermembranen (21) ($A_{tot} = N\pi d_B L$), $\varepsilon_M$ Membranporosität der im Hämodialysator (10) vorhandenen Hohlfasermembranen (21) ;

$P_{B,out}$ Blutdruck am Ausgang des Hämodialysators;

$P_{D,out}$ Dialysatdruck am Ausgang des Hämodialysators;

$\Pi_0$ mittlerer onkotischer Druck;

$x_i$ Lage des Inversionspunktes ($J_v(x_i) = 0$);

$\mu_B$ Blutviskosität;

$Q_B$ Blutflussrate;

L effektive Länge der im Hämodialysator (10) vorhandenen Hohlfasermembranen (21);

$\nu_1$ $J_v(0)$;

$\nu_2$ $J_v(L)$;

$\mu_D$ Viskosität des Dialysats;

$Q_D$ Dialysatflussrate;

$f_{R_o,R_k}$

$$\frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right),$$

$$R_o = \frac{d_B}{2} + \delta_M,$$

$$R_k = R_o + \delta_\varepsilon,$$

$d_B$ Innendurchmesser der im Hämodialysator (10) vorhandenen Hohlfasermembranen (21),

$\delta_M$ Wandstärke der im Hämodialysator (10) vorhandenen Hohlfasermembranen (21),

$\delta_\varepsilon$ Dicke der Diffusionsschicht um die im Hämodialysator (10) vorhandenen Hohlfasermembranen (21);

c. gegebenenfalls Bestimmen eines Gesamtvolumens $V_{tot}$ der Flüssigkeit, die während des Betriebs des Hämodialysators (10) durch die Wand der im Hämodialysator (10) vorhandenen Hohlfasermembranen (21) ausgetauscht wird, wobei das Gesamtvolumen $V_{tot}$ bestimmt wird gemäß

$$V_{tot} = \int_0^{TD} IFR(t)dt;$$

d. Übertragen der ermittelten internen Filtrationsrate IFR und gegebenenfalls des Gesamtvolumens $V_{tot}$ der ausgetauschten Flüssigkeit an eine Ausgabevorrichtung in operativer Verbindung mit dem Prozessor.

**Revendications**

**1.** Procédé mis en œuvre par un ordinateur pour déterminer un taux de filtration interne IFR dans un hémodialyseur capillaire (10) en utilisant les dimensions de l'hémodialyseur, les dimensions et les paramètres physiques de membranes à fibres creuses présentes dans l'hémodialyseur, et débits de sang et de dialysat à travers l'hémodialyseur, le procédé comprenant

i) acquérir, à l'aide d'un ordinateur, des données sur les propriétés physiques de l'hémodialyseur (10) et des membranes à fibres creuses (21) présentes dans l'hémodialyseur (10), les données comprenant un diamètre $d_H$ du boîtier de l'hémodialyseur (10), un nombre N de membranes à fibres creuses (21) présentes dans l'hémodialyseur (10), une longueur effective L des membranes à fibres creuses (21) présentes dans l'hémodialyseur (10), une surface totale $A_{tot}$ des membranes à fibres creuses (21) présentes dans l'hémodialyseur (10), un diamètre interne $d_B$ des membranes à fibres creuses (21) présentes dans l'hémodialyseur (10), une épaisseur de paroi $\delta_M$ des membranes à fibres creuses (21) présentes dans l'hémodialyseur (10), une porosité $\varepsilon_M$ des membranes à fibres creuses (21) présentes dans l'hémodialyseur (10), et un coefficient d'ultrafiltration $K_{UF}$ des membranes à fibres creuses (21) présentes dans l'hémodialyseur (10) ;

ii) acquérir, à l'aide de l'ordinateur, un flux sanguin $Q_B$ et un flux de dialysat $Q_D$ par l'intermédiaire de l'hémodialyseur (10) préalablement stocké en rapport avec une opération antérieure de l'hémodialyseur (10) ;

iii) déterminer, à l'aide de l'ordinateur, le débit de filtration interne IFR de l'hémodialyseur (10), sur la base des données acquises aux étapes i et ii, dans lequel le débit de filtration interne IFR est déterminé selon :

$$IFR = N\pi d_B K \left\{ \left( P_{B,out} - P_{D,out} - \pi_0 \right) x_i \right.$$

$$- \frac{128\mu_B}{N\pi d_B^4} \left[ Q_B \left( \frac{x_i^2}{2} - Lx_i \right) \right.$$

$$\left. - N\pi d_B \left[ \frac{\nu_2 - \nu_1}{6L} \left( \frac{x_i^4}{4} - L^3 x_i \right) + \frac{\nu_1}{2} \left( \frac{x_i^3}{3} - L^2 x_i \right) \right] \right]$$

$$\left. + \frac{\mu_D}{2N\pi f_{R_o,R_k}} \left[ Q_D \frac{x_i^2}{2} - N\pi d_B \left[ \frac{\nu_2 - \nu_1}{2L} \left( \frac{x_i^4}{12} - \frac{L^2}{2} x_i^2 \right) + \nu_1 \left( \frac{x_i^3}{6} - \frac{L}{2} x_i^2 \right) \right] \right] \right\}$$

avec

N nombre de membranes à fibres creuses (21) présentes dans l'hémodialyseur (10);

$d_B$ diamètre interne des membranes à fibres creuses (21) présentes dans l'hémodialyseur (10);

$$K^{\frac{K_{UF}}{A_{tot}\,\varepsilon_M}} ,$$

$K_{UF}$ Coefficient d'ultrafiltration des membranes à fibres creuses (21) présentes dans l'hémodialyseur (10),

$A_{tot}$ surface totale des membranes à fibres creuses (21) présentes dans l'hémodialyseur (10) ($A_{tot} = N\pi d_B L$),

$\varepsilon_M$ porosité des membranes à fibres creuses (21) présentes dans l'hémodialyseur (10) ;

$P_{B,out}$ pression sanguine à la sortie de l'hémodialyseur;

$P_{D,out}$ pression de dialysat à la sortie de l'hémodialyseur;

$\Pi_0$ pression oncotique moyenne;

$x_i$ emplacement du point d'inversion ($J_v(x_i) = 0$);

$\mu_B$ viscosité du sang;

$Q_B$ débit sanguin;

L longueur effective des membranes de fibres creuses (21) présentes dans l'hémodialyseur (10) ;

$v_1$ $J_v(0)$;

$v_2$ $J_v(L)$;

$\mu_D$ viscosité du dialysat;

$Q_D$ débit de dialysat;

$fR_o, R_k$

$$\frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right) ,$$

$$R_o = \frac{d_B}{2} + \delta_M ,$$

$$R_k = R_o + \delta_\varepsilon ,$$

$d_B$ diamètre interne des membranes à fibres creuses (21) présentes dans l'hémodialyseur (10),

$\delta_M$ épaisseur de paroi des membranes à fibres creuses (21) présentes dans l'hémodialyseur (10),

$\delta_\varepsilon$ épaisseur de la couche de diffusion autour des membranes à fibres creuses (21) présentes dans l'hémodialyseur (10).

2. Procédé selon la revendication 1, comprenant en outre

iv) acquérir, à l'aide de l'ordinateur, des données sur une durée TD du fonctionnement de l'hémodialyseur (10) ;

v) déterminer, à l'aide de l'ordinateur, un volume total $V_{tot}$ de fluide échangé à travers la paroi (22) des membranes à fibres creuses (21) présentes dans l'hémodialyseur (10) pendant le fonctionnement de l'hémodialyseur (10), sur la base du débit de filtration interne IFR et les données acquises à l'étape iv, dans lesquelles le volume total $V_{tot}$ est déterminé en fonction de

$$V_{tot} = \int_0^{TD} IFR(t)\,dt$$

3. Procédé selon la revendication 1 ou 2, dans lequel les données relatives aux propriétés physiques de l'hémodialyseur (10) et des membranes à fibres creuses (21) présentes dans l'hémodialyseur (10) sont acquises à partir d'une base de données en association opérative avec un processeur de l'ordinateur.

4. Procédé selon l'une quelconque des revendications 1 à 3, dans lequel des données relatives aux propriétés physiques de l'hémodialyseur (10) et des membranes à fibres creuses (21) présentes dans l'hémodialyseur (10) et/ou le débit sanguin et le débit de dialyse à travers l'hémodialyseur (10) pendant le fonctionnement de l'hémodialyseur (10) sont acquises par un dispositif de saisie en association opérationnelle avec un processeur de l'ordinateur.

5. Procédé selon la revendication 4, dans lequel le dispositif de saisie comprend un lecteur sans contact.

6. Procédé selon la revendication 4 ou 5, dans lequel le dispositif de saisie comprend au moins une interface utilisateur.

7. Procédé selon l'une quelconque des revendications 4 à 6, dans lequel le dispositif de saisie comprend un appareil de traitement sanguin extracorporel.

8. Un système comprenant

a) une base de données comprenant des données sur les propriétés physiques d'une pluralité d'hémodialyseurs capillaires (10) et de membranes à fibres creuses (21) présentes dans ceux-ci, les données comprenant un diamètre $d_H$ du boîtier des hémodialyseurs (10), un nombre N de membranes à fibres creuses (21) présentes dans les hémodialyseurs (10), une longueur effective L des membranes à fibres creuses (21) présentes dans les hémodialyseurs (10), une surface totale $A_{tot}$ des membranes à fibres creuses (21) présentes dans les hémodialyseurs (10), un diamètre interne $d_B$ des membranes à fibres creuses (21) présentes dans les hémodialyseurs (10), une épaisseur de paroi $\delta_M$ des membranes à fibres creuses (21) présentes dans les hémodialyseurs (10), une porosité $\varepsilon_M$ des membranes à fibres creuses (21) présentes dans les hémodialyseurs (10), et un coefficient d'ultrafiltration $K_{UF}$ des membranes à fibres creuses (21) présentes dans les hémodialyseurs (10); et/ou

b) un dispositif de saisie configuré pour fournir des données sur les propriétés physiques d'un hémodialyseur capillaire (10) et des membranes à fibres creuses (21) présentes dans l'hémodialyseur (10), les données comprenant un diamètre $d_H$ du boîtier de l'hémodialyseur (10), un nombre N des membranes à fibres creuses (21) présentes dans l'hémodialyseur (10), une longueur effective L des membranes de fibres creuses (21) présentes dans l'hémodialyseur (10), une surface totale $A_{tot}$ des membranes de fibres creuses (21) présentes dans l'hémodialyseur (10), un diamètre interne $d_B$ des membranes à fibres creuses (21) présentes dans l'hémodialyseur (10), une épaisseur de paroi $\delta_M$ des membranes à fibres creuses (21) présentes dans l'hémodialyseur (10), une porosité $\varepsilon_M$ des membranes à fibres creuses (21) présentes dans l'hémodialyseur (10), et un coefficient d'ultrafiltration $K_{UF}$ des membranes à fibres creuses (21) présentes dans l'hémodialyseur (10); et/ou pour fournir un débit sanguin et un débit de dialysat à travers l'hémodialyseur (10) pendant le fonctionnement de l'hémodialyseur (10);

c) un dispositif de sortie configuré pour la sortie de données reçues d'un processeur d'ordinateur en association opérationnelle avec le dispositif de sortie ;

d) un processeur d'ordinateur programmé pour la communication avec la base de données et/ou le dispositif de saisie, et pour la communication avec le dispositif de sortie, le processeur programmé pour

a. l'acquisition de données à partir de la base de données et/ou du dispositif de saisie, les données comprenant un diamètre $d_H$ du boîtier d'un hémodialyseur (10), un nombre N des membranes à fibres creuses (21) présentes dans l'hémodialyseur (10), une longueur effective L des membranes à fibres creuses (21) présentes dans l'hémodialyseur (10), une surface totale $A_{tot}$ des membranes à fibres creuses (21) présentes dans l'hémodialyseur (10), un diamètre interne $d_B$ des membranes à fibres creuses (21) présentes dans l'hémodialyseur (10), une épaisseur de paroi $\delta_M$ des membranes à fibres creuses (21) présentes dans l'hémodialyseur (10), une porosité $\varepsilon_M$ des membranes à fibres creuses (21) présentes dans l'hémodialyseur (10), et un coefficient d'ultrafiltration $K_{UF}$ des membranes à fibres creuses (21) présentes dans l'hémodialyseur (10); et/ou un débit sanguin et un débit de dialysat à travers l'hémodialyseur (10) pendant le fonctionnement de l'hémodialyseur (10),

b. déterminer un taux de filtration interne IFR dans un hémodialyseur capillaire (10), sur la base des données acquises, dans lequel le taux de filtration interne IFR est déterminé selon

$$IFR = N\pi d_B\, K \Bigg\{ \left(P_{B,out} - P_{D,out} - \pi_0\right)x_i$$

$$- \frac{128\mu_B}{N\pi d_B^4}\left[Q_B\left(\frac{x_i^2}{2} - Lx_i\right)\right.$$

$$\left.- N\pi d_B\left[\frac{v_2 - v_1}{6L}\left(\frac{x_i^4}{4} - L^3 x_i\right) + \frac{v_1}{2}\left(\frac{x_i^3}{3} - L^2 x_i\right)\right]\right]$$

$$+ \frac{\mu_D}{2N\pi f_{R_o,R_k}}\left[Q_D\,\frac{x_i^2}{2} - N\pi d_B\left[\frac{v_2 - v_1}{2L}\left(\frac{x_i^4}{12} - \frac{L^2}{2}x_i^2\right) + v_1\left(\frac{x_i^3}{6} - \frac{L}{2}x_i^2\right)\right]\right]\Bigg\}$$

avec

N nombre de membranes à fibres creuses (21) présentes dans l'hémodialyseur (10);

$d_B$ diamètre interne des membranes à fibres creuses (21) présentes dans l'hémodialyseur (10);

$K\ \dfrac{K_{UF}}{A_{tot}\,\varepsilon_M}$ , $K_{UF}$ Coefficient d'ultrafiltration des membranes à fibres creuses (21) présentes dans l'hémodialyseur (10), $A_{tot}$ surface totale des membranes à fibres creuses (21) présentes dans l'hémodialyseur (10) ($A_{tot} = N\pi d_B L$), $\varepsilon_M$ porosité des membrane à fibres creuses (21) présentes dans l'hémodialyseur (10);

$P_{B,out}$ pression sanguine à la sortie de l'hémodialyseur;

$P_{D,out}$ pression de dialysat à la sortie de l'hémodialyseur;

$\Pi_0$ pression oncotique moyenne;

$x_i$ emplacement du point d'inversion ($J_v(x_i) = 0$) ;

$\mu_B$ viscosité du sang;

$Q_B$ débit sanguin;

L longueur effective des membranes à fibres creuses (21) présentes dans l'hémodialyseur (10);

$v_1\ J_v(0)$;

$v_2\ J_v(L)$;

$\mu_D$ viscosité du dialysat;

$Q_D$ débit de dialysat ;

$f_{R_o,R_k}$

$$\frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right),$$

$$R_o = \frac{d_B}{2} + \delta_M,$$

$$R_k = R_o + \delta_\varepsilon,$$

$d_B$ diamètre interne des membranes à fibres creuses (21) présentes dans l'hémodialyseur (10),

$\delta_M$ épaisseur de paroi des membranes à fibres creuses (21) présentes dans l'hémodialyseur (10),

$\delta_\varepsilon$ épaisseur de la couche de diffusion autour des membranes à fibres creuses (21) présentes dans l'hémodialyseur (10),

c. éventuellement, déterminer un volume total $V_{tot}$ de fluide échangé à travers la paroi des membranes à fibres creuses (21) présentes dans l'hémodialyseur (10) pendant le fonctionnement de l'hémodialyseur (10), dans laquelle le volume total $V_{tot}$ est déterminé selon

$$V_{tot} = \int_0^{TD} IFR(t)dt;$$

d. transmettre le débit de filtration interne IFR déterminé et, éventuellement, le volume total $V_{tot}$ de fluide échangé vers le dispositif de sortie.

9. Système selon la revendication 8, dans lequel le dispositif de saisie, le dispositif de sortie et le processeur sont contenus dans un dispositif de communication mobile.

10. Système selon la revendication 8 ou 9, dans lequel le dispositif de saisie est une interface utilisateur graphique (GUI) d'un dispositif de communication mobile.

11. Système selon l'une quelconque des revendications 8 à 10, dans lequel le dispositif de sortie est un écran.

12. Programme d'ordinateur qui, lorsqu'il est exécuté sur un système de traitement de données, demande à un processeur d'ordinateur d'exécuter la méthode de

a. acquérir des données d'une base de données et/ou d'un dispositif de saisie en association opérationnelle avec le processeur, les données comprenant un diamètre $d_H$ du boîtier d'un hémodialyseur (10), un nombre N des membranes à fibres creuses (21) présentes dans l'hémodialyseur (10), une longueur effective L des membranes à fibres creuses (21) présentes dans l'hémodialyseur (10), une surface totale $A_{tot}$ des membranes à fibres creuses (21) présentes dans l'hémodialyseur (10), un diamètre interne $d_B$ des membranes à fibres creuses (21) présentes dans l'hémodialyseur (10), une épaisseur de paroi $\delta_M$ des membranes à fibres creuses (21) présentes dans l'hémodialyseur (10), une porosité $\varepsilon_M$ des membranes à fibres creuses (21) présentes dans l'hémodialyseur (10), et un coefficient d'ultrafiltration $K_{UF}$ des membranes à fibres creuses (21) présentes dans l'hémodialyseur (10); et/ou un débit sanguin et un débit de dialyse à travers l'hémodialyseur (10) pendant le fonctionnement de l'hémodialyseur (10);
b. déterminer un taux de filtration interne IFR dans un hémodialyseur capillaire (10), sur la base des données acquises, dans lequel le débit de filtration interne IFR est déterminé selon

$$IFR = N\pi d_B K \Bigg\{ \left( P_{B,out} - P_{D,out} - \pi_0 \right)x_i$$

$$- \frac{128\mu_B}{N\pi d_B^4}\left[ Q_B\left( \frac{x_i^2}{2} - Lx_i \right) \right.$$

$$- N\pi d_B\left[ \frac{\nu_2 - \nu_1}{6L}\left( \frac{x_i^4}{4} - L^3 x_i \right) + \frac{\nu_1}{2}\left( \frac{x_i^3}{3} - L^2 x_i \right) \right]\Bigg]$$

$$+ \frac{\mu_D}{2N\pi f_{R_o,R_k}}\left[ Q_D\frac{x_i^2}{2} - N\pi d_B\left[ \frac{\nu_2 - \nu_1}{2L}\left( \frac{x_i^4}{12} - \frac{L^2}{2}x_i^2 \right) + \nu_1\left( \frac{x_i^3}{6} - \frac{L}{2}x_i^2 \right) \right]\Bigg]\Bigg\}$$

avec

N nombre de membranes à fibres creuses (21) présentes dans l'hémodialyseur (10);
$d_B$ diamètre interne des membranes à fibres creuses (21) présentes dans l'hémodialyseur (10);

$K$ $\dfrac{K_{UF}}{A_{tot}\,\varepsilon_M}$ ,

$K_{UF}$ Coefficient d'ultrafiltration des membranes à fibres creuses (21) présentes dans l'hémodialyseur (10),
$A_{tot}$ surface totale des membranes à fibres creuses (21) présentes dans l'hémodialyseur (10) ($A_{tot} = N\pi d_B L$),
$\varepsilon_M$ porosité des membranes à fibres creuses (21) présentes dans l'hémodialyseur (10);
$P_{B,out}$ pression sanguine à la sortie de l'hémo-dialyseur ;
$P_{D,out}$ pression de dialysat à la sortie de l'hémodialyseur;

$\Pi_0$ pression oncotique moyenne;

$x_i$ emplacement du point d'inversion $(J_v(x_i) = 0)$ ;

$\mu_B$ viscosité du sang;

$Q_B$ débit sanguin;

L longueur effective des membranes à fibres creuses (21) présentes dans l'hémodialyseur (10);

$v_1$ $J_v(0)$ ;

$v_2$ $J_v(L)$ ;

$\mu_D$ viscosité du dialysat;

$Q_D$ débit de dialysat ;

$$f_{R_o,R_k} \quad \frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}(R_k^2 - R_o^2),$$

$$R_o = \frac{d_B}{2} + \delta_M,$$

$$R_k = R_o + \delta_\varepsilon,$$

$d_B$ diamètre interne des membranes à fibres creuses (21) présentes dans l'hémodialyseur (10),

$\delta_M$ épaisseur de paroi des membranes à fibres creuses (21) présentes dans l'hémodialyseur (10),

$\delta_\varepsilon$ épaisseur de la couche de diffusion autour des membranes à fibres creuses (21) présentes dans l'hémodialyseur (10) ;

c. éventuellement, déterminer un volume total $V_{tot}$ de fluide échangé à travers la paroi des membranes à fibres creuses (21) présentes dans l'hémodialyseur (10) pendant le fonctionnement de l'hémodialyseur (10), dans laquelle le volume total $V_{tot}$ est déterminé selon

$$V_{tot} = \int_0^{TD} IFR(t)dt;$$

d. transmettre le débit de filtration interne IFR déterminé et, éventuellement, le volume total $V_{tot}$ de fluide échangé vers un dispositif de sortie en association opérationnelle avec le processeur.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3102312 B1

- EP 3102314 B1

**Non-patent literature cited in the description**

- **LEGALLAIS, C. et al.** A theoretical model to predict the in vitro performance of hemodiafilters. *J. Membr. Sci.,* 2000, vol. 168, 3-15
- **A. LORENZIN et al.** Quantification of internal filtration in hollow fiber hemodialyzers with Medium cut-off membrane. *Blood Purif.,* 2018, vol. 46, 196-204

- **D. SCHNEDITZ et al.** Internal filtration, filtration fraction, and blood flow resistance in high- and low-flux dialyzers. *Clin. Hemorheol. Microcirc.,* 2014, vol. 58, 455-469
- **A. LORENZIN et al.** Quantification of Internal Filtration in Hollow Fiber Hemodialyzers with Medium Cut-off Membrane. *Blood Purif.,* 2018, vol. 46, 196-204